# EUROPÄISCHE PATENTSCHRIFT

(11) **EP 4 225 318 B1**
(45) Veröffentlichungstag und Bekanntmachung des Hinweises auf die Patenterteilung: **13.11.2024**
(21) Anmeldenummer: 21798288.3
(22) Anmeldetag: 07.10.2021
(51) Int. Cl.: A61K 31/513, A61K 31/519, A61P 25/28, A61P 9/00, A61K 45/06, A61K 31/517

(54) **MAGNESIUMOROTAT IN KOMBINATION MIT FOLSÄURE ZUR BEHANDLUNG VON DEMENZ**
MAGNESIUM OROTATE IN COMBINATION WITH FOLIC ACID FOR THE TREATMENT OF DEMENTIA
L'OROTATE DE MAGNÉSIUM EN COMBINAISON AVEC L'ACIDE FOLIQUE POUR LE TRAITEMENT DE LA DÉMENCE

(30) Priorität: 07.10.2020 EP 20200644
(43) Veröffentlichungstag der Anmeldung: 16.08.2023
(73) Patentinhaber: WÖRWAG Pharma GmbH & Co.KG, 71034 Böblingen (DE)
(72) Erfinder: WÖRWAG, Fritz, 70192 Stuttgart (DE); WÖRWAG, Marcus, 70469 Stuttgart (DE)
(74) Vertreter: Simandi, Claus
(86) Internationale Anmeldenummer: PCT/EP2021/077684
(87) Internationale Veröffentlichungsnummer: WO 2022/074114

(56) Entgegenhaltungen:
- WO-A2-2010/145644
- DE-U1- 202017 006 840
- US-B1- 6 420 342
- BUCK G: "NICHT NUR MAGNESIUM, AUCH OROTSAEURE HILFT BEI KARIOLOGISCHEN PROBLEMEN", ZFA. ZEITSCHRIFT FUER ALLGEMEINMEDIZIN, HIPPOKRATES VERLAG, STUTTGART, DE, vol. 68, no. 12, 30 April 1992 (1992-04-30), pages 396, XP000609779, ISSN: 0341-9835
- MA FEI ET AL: "Folic acid supplementation improves cognitive function by reducing the levels of peripheral inflammatory cytokines in elderly Chinese subjects with MCI", vol. 6, no. 1, 23 November 2016 (2016-11-23), XP055787301, Retrieved from the Internet <URL:http://www.nature.com/articles/srep37486> DOI: 10.1038/srep37486
- "Orotic Acid, 1st edition", 1 January 1980, MTP PRESS LIMITED, ISBN: 978-94-009-8045-7, article CIHÁK ALOIS ET AL: "Chapter 8 - Physiological Effects", pages: 36 - 36, XP055787619, DOI: 10.1007/ 978-94-009-8043-3

## Beschreibung

Die Erfindung betrifft ein Mittel zur Behandlung und Prophylaxe von Demenz (erkrankungen), insbesondere ein Arznei- oder Nahrungsergänzungsmittel sowie dessen Verwendung, welche eine Kombination von Biofaktoren enthält.

Die Demenz wird in der ICD-10 (Internationale statistische Klassifikation der Krankheiten und verwandter Gesundheitsprobleme, 10. Revision (2019)) der Weltgesundheitsorganisation (WHO) in Kapitel V "Psychische und Verhaltensstörungen", F00-F03 klassifiziert und ist ein Syndrom als Folge einer meist chronischen oder fortschreitenden Krankheit des Gehirns mit Störung vieler höherer kortikaler Funktionen, einschließlich Gedächtnis, Denken, Orientierung, Auffassung, Rechnen, Lernfähigkeit, Sprache und Urteilsvermögen. Das Bewusstsein ist nicht getrübt. Die kognitiven Beeinträchtigungen werden gewöhnlich von Veränderungen der emotionalen Kontrolle, des Sozialverhaltens oder der Motivation begleitet. Dieses Syndrom kommt bei Alzheimer-Krankheit, bei zerebrovaskulären Störungen und bei anderen Zustandsbildern vor, die primär (unmittelbar) oder sekundär (mittelbar) das Gehirn betreffen (siehe auch "Dementia: A Public Health Priority" (2012) (https://www.alz.co.uk/WHO-dementia-report)).

Die Alzheimer(sche) Demenz (AD) ist die häufigste Demenzform und betrifft heute mehr als 60 % der demenzkranken Menschen weltweit. Pathologisches Hauptmerkmal der AD ist die Bildung von senilen oder amyloiden Plaques, bestehend aus dem Aß-Peptid, und neurofibrillären Aggregaten einhergehend mit synaptischer und neuronaler Degeneration und Gliose, wobei hyperphosphorylierte Mikrotubuli mit dem Tau-Protein (MAPT) assoziiert sind. Das Aß-Peptid entsteht durch die Aktivitäten mindestens zweier verschiedener Proteasen aus einem Vorläuferprotein, dem "Amyloid Precursor Protein" (APP), welches in der Zellwand von Neuronen lokalisiert ist. Bei dem proteolytischen Abbau von APP und durch nachträgliche Modifikation entstehen Aß-Fragmente unterschiedlicher Länge und Art. Die Ablagerung von Aß in Form von Plaques werden nach wie vor als zumindest ein Auslöser der Krankheitssymptome angesehen. Bei der vaskulären (gefäßbedingten) Demenz führen Durchblutungsstörungen im Gehirn zum Absterben von Neuronen. Sie können zum Beispiel die Folge von mehreren kleinen Schlaganfällen (z.B. infolge Gefäßverschlusses) sein, die ebenfalls zeitlich versetzt auftreten (sog. "Multi-Infarkt-Demenz") können.

Bisher können allenfalls die Symptome einer Demenz wie AD, welche infolge des Absterbens von Neuronen entstehen und den Grad einer Demenz bestimmen, symptomatisch, aber nicht kausal, behandelt werden.

Es sind keine zugelassenen Medikamente bis heute bekannt, die die Krankheitsprozesse bei einer (primären) Demenz mit einer ursächlichen Erkrankung des Gehirns heilen können.

Der Krankheitsverlauf oder gar der Eintritt der Demenz kann jedoch verzögert oder positiv beeinflusst werden. Neben der Verringerung des individuellen Risikos (z.B. Vermeidung von Übergewicht (Adipositas), mangelnde Bewegung, Rauchen, Alkohol, negativer Stress, etc.) können Biofaktoren, wie Vitamine u.a. einen wirksamen Beitrag leisten, wobei eine langfristige und kontinuierliche Behandlung oder Prophylaxe verfolgt werden soll. Dies ist Gegenstand der Forschung der Anmelderin.

Die AD ist eine multifaktorielle Krankheit, bei der die Rolle von metabolischen und entzündlichen Begleiterkrankungen zunehmend wichtiger zu werden scheint. Nur ~2 % der AD-Fälle werden dabei durch somatische Mutationen im Amyloidvorläuferprotein (APP), Presenilin 1 (Psen 1) und Presenilin 2 (Psen 2) verursacht (familiale AD, fAD), während 98 % der AD-Fälle eine sporadische (sporadische AD, sAD), noch unbekannte, aber komplexe und multifaktorielle Ätiologie haben [1-3]. Genomweite Assoziationsstudien (GWAS) haben bisher Allele des Apolipoproteins E, genauer ApoE ε4, mit einem erhöhten Risiko für die Entwicklung von sowohl milder kognitiver Beeinträchtigung als auch AD in Verbindung gebracht [4, 5]. Andere genetische Faktoren, die aufgrund von GWAS erkannt wurden und die mit dem Auftreten von sAD in Verbindung gebracht werden, werden z.B. von Apolipoprotein J (APOJ), Phosphatidylinositol bindendem Clathrin-Assemblierungsprotein (PICALM), von myeloischen Zellen exprimiertem Trigger-Rezeptor 2 (TREM2), Differenzierungscluster 33 (CD33) und Komplementrezeptor 1 (CR1) [4-7] repräsentiert. Seit Kurzem wird metabolischen Begleiterkrankungen von AD, die durch Mangelernährung, insbesondere in westlichen Ländern, verursacht wird, eine zunehmend größere Rolle zugeschrieben [8, 9]. Eine der Begleiterkrankungen von sAD, die bisher identifiziert wurde, ist Diabetes (Typ I oder Typ II), ein unter Bevölkerungsgruppen der westlichen Welt weit verbreiteter pathologischer Zustand, wobei schätzungsweise derzeit 176 Millionen Menschen weltweit von Typ I (-5-10 % der Fälle), von Typ II (~90 % der Fälle) oder anderen Formen von Diabetes, z.B. Schwangerschaftsdiabetes, betroffen sind [9, 10]. Diabetes Typ II, der weltweit für den größten Teil der Diabetesfälle verantwortlich ist, tritt verstärkt bei älteren Menschen auf und wird mit energiereicher, minderwertiger Ernährung im Rahmen einer bewegungsarmen Lebensweise in Verbindung gebracht - Umstände, die in den Bevölkerungsgruppen westlicher Industriestaaten häufig anzutreffen sind. Die Ernährungsweise in solchen Ländern beinhaltet häufig billige, sehr stark verarbeitete Lebensmittel, die einen erheblichen Anteil an gesättigten Fettsäuren, Cholesterol und einfachen Kohlenhydraten enthalten und denen gleichzeitig essentielle Mineralstoffe, Spurenelemente und Vitamine fehlen [11-14]; z.B. besteht in westlichen Bevölkerungsgruppen und insbesondere unter älteren Menschen oft ein chronischer Mangel insbesondere an Magnesium, Zink und Vitamin B, insbesondere Thiamin, Folsäure, oder Prodrugs wie Benfotiamin und Vitamin D, wobei ein Mangel alle Stadien, von asymptomatischen - lediglich durch Laborbestimmungen feststellbaren - Stadien bis hin zu klinisch manifesten Mangelzuständen mit oder ohne spezifischen Symptomen reichen kann. Es fällt auf, dass ~80 % der AD-Patienten im Verlauf der Demenz eine Glukoseunverträglichkeit oder eine Diabetes entwickeln [15, 16], was auf eine gegenseitige Wechselwirkung hinweist [17]. Damit einhergehend ist eine mitochondriale Dysfunktion zu sehen, welche (mit-)ursächlich für eine Demenz sein kann.

Zudem führen westliche Ernährungsweisen in Kombination mit mangelnder körperlicher Aktivität häufig zu Adipositas [14], und insbesondere eine erhöhte Anreicherung von Viszeral-Fett verursacht eine Metaflammation [18] (sogenannte "kalte Entzündung"), die zu einer Freisetzung entzündungsfördernder Cytokine IL1β und TNF-α aus infiltrierenden Makrophagen und Hepatozyten führt und somit eine systemische metabolische Verschlechterung erfolgt [18, 19]. Sowohl Adipositas als auch Diabetes verschlimmern bzw. "befeuern" die Pathologie von AD [9, 11, 20].

Angesichts der Tatsache, dass in einer insgesamt älter werdenden Bevölkerung immer mehr Patienten an Stoffwechselstörungen, einschließlich von Diabetes und Adipositas (die in erster Linie durch Fehlernährung allein oder in Kombination mit Bewegungsmangel verursacht werden) leiden, die einen Hauptrisikofaktor für die Entwicklung einer Demenz, insbesondere AD darstellen, sind insbesondere ein Mangel oder verminderte Zufuhr an Biofaktoren, insbesondere Magnesium, Zink und Vitamin B, insbesondere Thiamin, Folsäure, oder Prodrugs wie Benfotiamin und Vitamin D, für ältere Menschen risikoreich. Dies geht einher mit veränderten Stoffwechselprozessen älterer Menschen (z.B. Muskelschwund, etc.).

Biofaktoren zur Behandlung und Prophylaxe einer Demenz sind im Stand der Technik beschrieben. Beispielsweise kann bereits ein Vitaminmangel, insbesondere ein Mangel an Vitamin B12 eine (sekundäre) Demenz verursachen (WHO ICD10 (2019), F02.8).

Im Fokus der Anmelderin stehen, wie vorstehend erläutert, insbesondere folgende Verbindungen oder Stoffe, und zwar Magnesium- oder Zinkorotat, Vitamin D, insbesondere Vitamin D3 (Cholecalcalciferol) und Vitamin B, insbesondere Thiamin, Folsäure, Vitamin B6 und B12 (Cobalamin) und Benfotiamin.

Insbesondere Orotate erweisen sich als vielversprechende Zn- und Mg Vehikel, da sie die Blut-Hirn-Schranke leicht überwinden können. Orotate sind zudem von Vorteil, da z.B. besonders sowohl Magnesiumorotat als auch Zinkorotat leicht durch die Zellmembran geführt/permeiert werden kann, und ebenfalls von Mitochondrien und dem Zellkern bevorzugt aufgenommen werden [21]. Eine daraus resultierende Anregung der Energiebereitstellung, mithin z.B. der ATP-Produktion in Mitochondrien ist vorteilhaft, da eine Demenz, insbesondere AD, (mit-)ursächlich eine mitochondriale Dysfunktion aufweisen kann (supra).

Weiterhin sind Kombinationspräparate von vorstehenden Biofaktoren zur Behandlung und Prophylaxe einer Demenz im Stand der Technik beschrieben.

So beschreibt US20150132273A1 Magnesium-Threonat in Kombination mit Biofaktoren zur Behandlung von kognitiven Beeinträchtigungen. Allerdings erweist sich Threonat als ineffizientes Vehikel für Magnesium oder Zink, da Threonat selbst in mehreren Stoffwechselwegen schnell abgebaut wird.

Der Einsatz von Magnesiumsalzen, jedoch nicht Magnesiumorotat, in Kombination mit Biofaktoren zur Demenzbehandlung wird in WO 2017/179644 A1 beschrieben.

WO 2017/059895 A1 offenbart in Beispiel 1 eine Zubereitung aus L-5-Methyltetrahydrofolat (100-800 ug Folsäureäquivalent) Vitamin B3 (4-40 mg), Vitamin B2 (0.4-5 mg), Methylcobalamin, (0.5-10 µg), Vitamin B6 (0.4-5 mg), Trimethylglycine (100-2000 mg), Zinkbisglycinat 5-50 mg) und N-Acetylcystein (100-2000 mg) zur Behandlung von Demenz.

Mischoulon et al [22] offenbart Folat zur Demenzbehandlung, einschließlich der senilen Demenzerkrankung nach Alzheimer und zur Prophylaxe derselben. Eine Gabe von 15 mg per Tag an Patienten wird beschrieben.

DE 20 2017 006840 U1 offenbart Nahrungsergänzungsmittel enthaltend 0,1 bis 3 g Zinkorotat-Dihydrat und 1 bis 15 mg Folsäure, und zwar in Form einer Brausetablette, eines Getränkes oder eines Pulvers für die Behandlung von Morbus Alzheimer, Demenz oder Fatigue-Syndrom, jedoch ist Magnesiumorotat nicht offenbart.

WO2010145644A2 offenbart Zusammensetzungen enthaltend Orotsäure mit einer Trägersubstanz, z.B. Folsäure zur Behandlung von Demenz, jedoch ist Magnesiumorotat nicht offenbart.

US6420342B1 beschreibt Nahrungsmittelzusammensetzungen enthaltend Folsäure und Orotat, z.B. Natriumorotat für die Behandlung von kardiologischen oder kardiovaskulären Erkrankungen, sowie von Alzheimer oder chronischer Ermüdung (Fatigue), jedoch ist Magnesiumorotat nicht offenbart.

BUCK G, "NICHT NUR MAGNESIUM, AUCH OROTSAEURE HILFT BEI KARIOLOGISCHEN PROBLEMEN", ZFA. ZEITSCHRIFT FUER ALLGEMEINMEDIZIN, HIPPOKRATES VERLAG, STUTTGART, vol. 68, no. 12, page 396, offenbart, dass nicht nur Magnesium, sondern auch Orotsäure bei kardiologischen Problemen wie beispielsweise chronischer Herzinsuffizienz hilft, und erwähnt positive klinische Ergebnisse mit Magnesiumorotat hinsichtlich dieser Erkrankungen, jedoch nicht Demenz.

Ma Fei et al. "Folie acid supplementation improves cognitive function by reducing the levels of peripheral inflammatory cytokines in elderly Chinese subjects with MCI", vol. 6, no. 1, 37486, SCIENTIFIC REPORTS, Nature (2016) offenbart, dass Supplementierung mit Folsäure die kognitive Funktion erhöht, jedoch ist Magnesiumorotat nicht offenbart.

Cihäk Alois et al. "Chapter 8 - Physiological Effects", Orotic Acid, 1st edition, MTP Press Limited, page 36, paragraph 3 (ISBN 978-94-009-8045-7) offenbart Orotsäure zur Behandlung von experimenteller Hepatitis in vivo, jedoch sind Magnesiumorotat und Folsäure nicht offenbart.

Ausgehend vom diesem Stand der Technik ist es daher Aufgabe der vorliegenden Erfindung, besonders geeignete und wirksame Kombinationen von Biofaktoren zur Behandlung und Prophylaxe einer Demenz bereitzustellen, insbesondere zur Langzeittherapie oder Langzeitprophylaxe.

Hierzu hat die Anmelderin umfangreiche Untersuchungen zu präklinischen AD-Modellen durchgeführt, welche die Eignung von Biofaktorenkombinationen zur Prophylaxe und Behandlung von Demenz, insbesondere AD, zeigt.

Die immortalisierten Neuroblastomazellen, und zwar humane SY-SY5Y-APP695 Zellen über-exprimieren das neuronale, humane APP-Gen, sodass eine verstärkte Bildung von β-Amyloid erfolgt.

Die Nematode C. Elegans (CL2006) exprimiert humanes Amyloid-β (1-42) unter der Kontrolle eines Muskel-spezifischen Promotors, sodass eine progressive Paralyse des Wurms erfolgt. Die Lebensspanne ist reduziert und der Wurm zeigt charakteristische Einlagerungen von β-Amyloid.

Alzheimer Ratten TgF344-AD wurden nach 6-7 und 15-16 Monaten auf Aktivität der oxidativen Phosphorylierung der Mitochondrien gegenüber Wildtypen geprüft.

Die Ergebnisse sind in den Beispielen und Figuren erläutert und ausgewiesen.

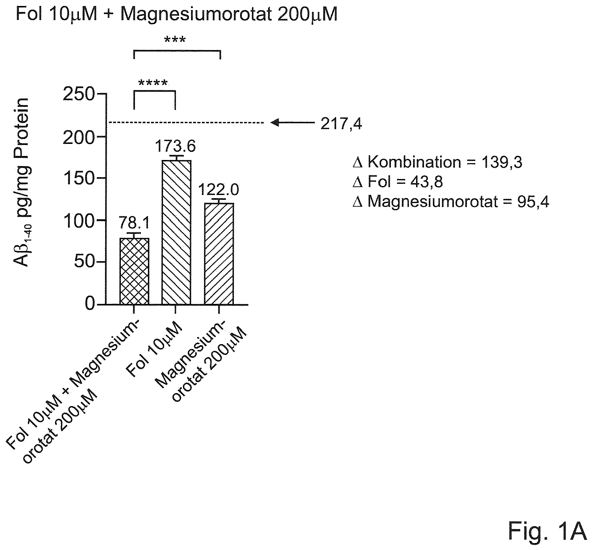

Überraschender Weise konnte für eine Zusammensetzung aus Magnesiumorotat und Folsäure in immortalisierten Neuroblastomazellen, und zwar humane SY-SY5Y-APP695 Zellen, eine überadditive Hemmung der Bildung von β-Amyloid erreicht werden (Figur 1A).

Dieses Ergebnis wird insbesondere auch in der Eignung dieser Kombination gesehen, die ATP-Produktion in den Mitochondrien zu normalisieren und einer ursächlichen mitochondrialen Dysfunktion entgegenzuwirken.

Daher betrifft die Erfindung ein Mittel, insbesondere Arzneimittel oder Nahrungsergänzungsmittel oder bilanzierte Diät, umfassend oder bestehend aus Magnesiumorotat und Folsäure zur Verwendung in der Prophylaxe und Behandlung von Demenz (nachstehend: erfindungsgemäßes Mittel).

Vorgenannte Untersuchungen zeigen, dass neben Magnesiumorotat und Folsäure insbesondere ein "Cocktail" aus Magnesium- oder Zinkorotat, Vitamin D, insbesondere Vitamin D3 (Cholecalcalciferol) und Vitamin B, insbesondere Thiamin, Folsäure, Vitamin B6 und B12 (Cobalamin) und Benfotiamin die Wirkung zumindest additiv unterstützen.

Daher sind ebenfalls weitere Kombinationen erfindungsgemäß umfasst, wobei mindestens zwei oder drei oder vier oder fünf oder sechs Substanzen oder sämtliche Substanzen ausgewählt sind aus der Gruppe Magnesiumorotat, Zinkorotat, Vitamin D3, Vitamin B6, Folsäure, Vitamin B12 und Benfotiamin.

Magnesiumorotat ist ein Salz der Orotsäure (bzw. 6-Carboxyuracil) mit der Formel 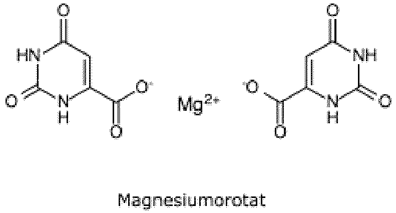 und kommerziell erhältlich, z.B. Wörwag Pharma GmbH (Böblingen, Deutschland).

Zinkorotat ist ein Salz der Orotsäure (bzw. 6-Carboxyuracil) mit der Formel 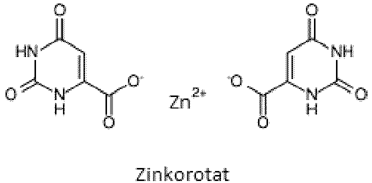 und kommerziell erhältlich, z.B. Wörwag Pharma GmbH (Böblingen, Deutschland).

Folsäure (bzw. Pteroyl-mono-glutaminsäure) ist Vitamin B9 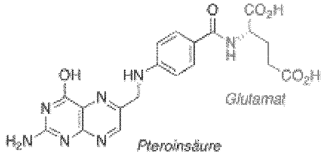 bzw. die Tautomere (2S)-2-[(4-[(2-Amino-4-oxo-1H-pteridin-6-yl)methylamino]benzoyl)amino]pentandisäure (Lactam) oder (2S)-N-(4-[([2-Amino-4-hydroxypteridin-6-yl]methyl)amino]benzoyl)glutaminsäure (Lactim) und können als Folate vorliegen, wobei 2-7 Glutamylreste vorliegen. Zudem können Folate am Pteridin hydriert sein, wie z.B. das 5, 6, 7, 8-Tetrahydrofolat oder substituiert (z.B. Methyl-, wie 5-Methyltetrahydrofolat u.a.) sein. Erfindungsgemäß sind solche Folate (bzw. Folsäureäquivalente) mit dem Begriff Folsäure mitumfasst. Ebenfalls erfindungsgemäß umfasst sind Tautomere und Salze der Folsäure. Die synthetisch hergestellte Folsäure ist erfindungsgemäß bevorzugt.

Benfotiamin ist ein lipophiles Prodrug des Thiamins (Vitamin B1) und stellt somit ein Provitamin dar und wird z.B. von der Anmelderin als milgamma protekt^{®} vertrieben, und zwar zur Behandlung von Neuropathien und kardiovaskulären Störungen, die durch Vitamin-B1-Mangel hervorgerufen werden.

In einer weiteren bevorzugten Ausführungsform des erfindungsgemäßen Mittels ist das molare Verhältnis von Magnesiumorotat zu Folsäure vorzugsweise 2-25:1, insbesondere 20:1.

Die Vitamine B und D betreffen die handelsüblichen Vitamine D2 und D3 und B1 bis B12.

Die genannten Stoffe können in ihren physiologisch aktiven Formen eingesetzt werden, Salze, Cofaktoren und Hydrate umfassen oder Stereoisomere, Tautomere (z.B. R, S, Z, E), etc.

Im Rahmen dieser Erfindung wird unter Demenz(erkrankung(en)) solche verstanden, nicht abschließend, wie eine Alzheimer Demenz bzw. Alzheimer-Krankheit, Demenz bei Alzheimer-Krankheit mit frühem Beginn (Typ 2), Demenz bei Alzheimer-Krankheit mit spätem Beginn (Typ 1), Demenz bei Alzheimer-Krankheit mit atypischer oder gemischter Form, Vaskuläre Demenz, Demenz bei Lewy-Körper-Krankheit, wobei alle diese Formen erfindungsgemäß umfasst sind. Diese Demenzformen sind in der "Internationalen statistischen Klassifikation der Krankheiten und verwandter Gesundheitsprobleme", 10. Revision (2019) der Weltgesundheitsorganisation (WHO) in Kapitel V "Psychische und Verhaltensstörungen" zu F00-03 (supra) beschrieben.

Sämtliche genannten Indikationen sind z.B. im Pschyrembel, 267. Auflage 2017, De Gruyter (Berlin) beschrieben.

Das wirkende Mittel enthaltend die erfindungsgemäße Kombination aus Magnesiumorotat und Folsäure, kann nunmehr vorteilhaft zur Behandlung und Prophylaxe eines erkrankten Patienten oder Individuums, Tier, Säugetier oder vorzugsweise Mensch, und zwar zur Behandlung und Prophylaxe von Demenz(erkrankungen) eingesetzt werden.

Die Mittel können in beliebigen Mengen und Dosierungen verabreicht werden.

Die galenische Formulierung des erfindungsgemäßen Mittels kann ausgewählt sein aus der Gruppe bestehend aus: Tropfen, Saft, Sirup, Tabletten, Dragees, Kapseln, Retard-Formulierungen, Infusionen, Salben, Emulsionen, Puder oder Pulver. Selbstverständlich kann die Formulierung pharmazeutisch übliche Hilfsstoffe enthalten.

In einer weiteren Ausführungsform betrifft die Erfindung ein Arzneimittel enthaltend ein erfindungsgemäßes Mittel zur Verwendung oder Anwendung zur Behandlung und Prophylaxe von Demenz(erkrankungen).

Eine weitere bevorzugte Ausführungsform betrifft ein Nahrungsergänzungsmittel enthaltend das erfindungsgemäße Mittel, insbesondere in Form einer diätischen Zusammensetzung oder bilanzierte Diät zur Behandlung und Prophylaxe von Demenz(erkrankungen). Geeignete erfindungsgemäße Nahrungs- oder Lebensmittel, einschließlich Wasser, sind solche wie z.B. in der Verordnung (EG) Nr. 178/2002 vom 28. Januar 2002 nicht abschließend definiert, solche wie Backwaren und Getränke und Kindernahrungszubereitungen. Das erfindungsgemäße Nahrungsergänzungsmittel kann mit einem geeigneten physiologisch verträglichen Träger versetzt werden.

Die erfindungsgemäßen pharmazeutischen Zubereitungen können in Form von Dosierungseinheiten hergestellt werden. Dies bedeutet, dass die Zubereitungen in Form einzelner Teile, vorzugsweise Kapseln und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge des erfindungsgemäßen Mittels, die bei einer Applikation verabreicht wird und die gewöhnlich einer ganzen, einer halben, einem Drittel oder einem Viertel einer Tagesdosis entspricht. Bevorzugt ist eine Dosierung von dreimal täglich, vorzugsweise in Form einer Tablette oder Tropfen, insbesondere morgens, mittags und abends, ggfs. zu den Mahlzeiten.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen, wie a) Füll- und Streckmittel, z.B. Stärken, Milchzucker, Rohrzucker, Glukose, Mannit, Dextrine, Maltodextrin und Kieselsäure, hochdisperses Siliciumdioxid, b) Bindemittel, z.B. Carboxymethylcellulose, Cellulosepulver, mikrokristalline Cellulose, Alginate, Gelatine, Polyvinylpyrrolidon, c) Feuchthaltemittel, z.B. Glycerin, d) Sprengmittel, z.B. Agar-Agar, Calciumcarbonat und Natriumcarbonat, e) Lösungsverzögerer, z.B. Paraffin und f) Resorptionsbeschleuniger, z.B. quartäre Ammoniumverbindungen, g) Netzmittel, z.B. Cetylalkohol, Glycerinmonostearat, h) Adsorptionsmittel, z.B. Kaolin und Bentonit und i) Gleitmittel, z.B. Talkum, Calcium- und Magnesiumstearat und feste Polyethylenglykole oder Gemische der unter a) bis i) aufgeführten Stoffe.

Die Tabletten, Dragees, Kapseln, Pillen und Granulate können mit den üblichen, gegebenenfalls Opakisierungsmittel enthaltenden Überzügen und Hüllen versehen sein, z.B. solche wie nicht abschließend Hypromellose, mikrokristalline Cellulose, Stearinsäure, Titandioxid, und ebenfalls so zusammengesetzt sein, dass sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

Daher betrifft die Erfindung ebenfalls eine pharmazeutische Zubereitung enthaltend ein erfindungsgemäßes Mittel samt Hilfs- und Zusatzstoffen.

### Beispiele und Figuren:

Nachfolgende Beispiele und Figuren dienen ausschließlich zur Erläuterung der Erfindung, ohne die Erfindung auf diese Beispiele zu beschränken.

### Beispiel 1:

### Zellmodell:

### Zellkultivierung:

Die verwendeten humanen Neuroblastom-SH-SY5Y-Zellen wurden mit DNA des humaner Wildtyp-APP695 (APP) stabil transfiziert (Grewal R, Reutzel M, Dilberger B et al. (2020) Purified oleocanthal and ligstroside protect against mitochondrial dysfunction in models of early Alzheimer's disease and brain ageing. Exp Neurol 328: 113248, Stockburger C, Gold VAM, Pallas T et al. (2014) A cell model for the initial phase of sporadic Alzheimer's disease. J Alzheimers Dis 42: 395-411).

SH-SY5Y-APP Zellen wurden bei 37°C unter einer Atmosphäre von 5% CO2 in Kulturmedium (DMEM) kultiviert, ergänzt mit 10% (v/v) hitzeinaktiviertem fetalem Kälberserum, 60 pg/ml Streptomycin, 60 Einheiten/ml Penicillin, 0,3 mg/ml Hygromycin, nicht-essentiellen MEM-Aminosäuren und 1 mM Natriumpyruvat 1%. Die Zellen wurden alle 3 Tage passagiert und verwendet, wenn sie 70 - 80% Konfluenz erreichten.

Die Zellen wurden mit 200µM Magnesiumorotat, 10µM Folsäure und deren Kombination für 24h inkubiert. Als Kontrolle diente eine Inkubation mit DMEM.

### Aβ 1-40 Messung:

Nach 24 h Inkubation wurde das Medium in den Zellkulturmediumflaschen gesammelt und die Zellen mit kalter PBS gespült. Die Suspension wurde anschließend 5 Minuten lang bei 220 g zentrifugiert. Danach wurde der Überstand verworfen und die Zellpellets in 1,5 ml PBS und Proteaseinhibitor resuspendiert. Die Lösung wurde dann 5 Minuten lang bei 112 g zentrifugiert und der Überstand wurde entfernt. Das Zellpellet wurde gesammelt und 600 µl Zellextraktionspuffer (Thermo Fisher Scientific, Waltham, MA, USA) hinzugefügt und für 30 Minuten inkubiert. Danach wurde die Suspension 10 Minuten lang bei 13.000 g zentrifugiert. Der Überstand wurde in ein neues Röhrchen überführt und bei -80°C gelagert. Die Überstände wurden auf Eis aufgetaut und in eine 384er Platte pipettiert (Greiner Bio-One, Kremsmünster, Österreich). Zum Nachweis der Amyloid-Beta-Konzentration wurde ein HTRF-Amyloid-Beta 1-40 Kit (Cisbio, Codolet, Frankreich) verwendet. Die Proben wurden nach den Anweisungen des Herstellers verwendet. Danach wurde die optische Dichte bei 665 und 622 nm gemessen. Die Amyloid-Beta Werte wurden anschließend auf den Proteingehalt normalisiert.

### Protein-uantifizierun:

Der Proteingehalt wurde mit dem PierceTM Protein Assay Kit (Thermo Fisher Scientific, Waltham, MA, USA) bestimmt. Die Anweisungen wurden wie vom Hersteller angegeben befolgt. Als Standard wurde Rinderserumalbumin verwendet. Das Protokoll basiert auf einer Veröffentlichung von Smith et al. (Smith PK, Krohn RI, Hermanson GT et al. (1985) Measurement of protein using bicinchoninic acid. Analytical Biochemistry 150: 76-85).

### Nematode (Caenorhabditis elegans)

### C. elegans und Bakterienstämme

Der C. elegans Wildtyp-Stamm N2 stammt vom Caenorhabditis elegans Genetics Center (Universität von Minnesota, MN, USA). Die Nematoden wurden auf Nematoden-Wachstumsmedium gehalten (NGM)-Agarplatten, die mit E. coli OP50 bei 20 °C nach Standardprotokollen (Brenner S (1974) The genetics of Caenorhabditis elegans. Genetics 77: 71-94) ausgesät wurden. Für alle Experimente wurden synchrone Populationen durch ein Standard-Bleichprotokoll generiert (Theresa Stiernagle (2006) Maintenance of C. elegans. WormBook: 1-11).

### Kultivierung und Behandlung:

Synchrone Larven wurden zweimal in M9 Puffer gewaschen, gezählt und auf 10 Larven pro 10 µL eingestellt. Die Nematoden wurden in 96-Well-Platten (Greiner Bio-One, Frickenhausen, Deutschland) pipettiert. Die Konzentration von OP50 wurde bei der optischen Dichte OD600 mit Flüssignematoden auf 1 eingestellt. OP50-NGM wurde als standardisierte Nahrungsquelle hinzugefügt, wobei das 4,4-fache des Volumens der Larven mit M9-Lösung verwendet wurde. Die L1-Larven wurden unter ständigem Schütteln bei 20 °C gehalten und erreichte innerhalb von 3 Tagen das Erwachsenenalter. Effektoren wurden nach Erreichen des jungen Erwachsenenalters 48 h vor dem Experiment hinzugefügt. Als Effektoren wurden 100µM Magnesium-Orotat und 50µM Folsäure in M9 gelöst aufgetragen. M9 Puffer wurde als Kontrolle aufgetragen.

### Heat-stress Resistance:

Etwa 10 Nematoden wurden, wie oben erwähnt, pro Vertiefung in einer 96-Well-Mikroplatte aufgezogen. Nach 48 Stunden Inkubation wurden, wie zuvor beschrieben, Effektoren aufgetragen. Die Zeit bis zum Tod der Nematoden wurde mit einem Mikroplatten Thermo Toleranztest bestimmt, wie er bei Fitzenberger et al. beschrieben ist (Fitzenberger E, Deusing DJ, Marx C et al. (2014) The polyphenol quercetin protects the mev-1 mutant of Caenorhabditis elegans from glucose-induced reduction of survival under heat-stress depending on SIR-2.1, DAF-12, and proteasomal activity. Molecular nutrition & food research 58: 984-994).

Die Nematoden wurden mit M9 Puffer in 15-mL-Röhrchen aus den Vertiefungen gewaschen, gefolgt von drei zusätzliche Waschschritte. In jeder Vertiefung einer schwarzen 384-Well-Mikrotiterplatte (Greiner Bio-One, Frickenhausen, Deutschland), wurden 6,5 µL M9 Puffer/Tween^{®} 20 (1% v/v) Lösung hinzugefügt. Anschließend wurde 1 µL M9-Puffer mit einem Nematoden unter einem Stereomikroskop (Breukhoven Microscope Systems, Capelle aan den Ijssel, Niederlande), in jede Vertiefung pipettiert und gemischt mit 7,5 µl SYTOXTM green (Endkonzentration 1 M; Life Technologies, Karlsruhe, Deutschland). Zur Verhinderung der Wasserverdunstung wurden die Platten mit einer Rotilab-Siegelfolie versiegelt (Greiner Bio-One, Frickenhausen, Deutschland). Es wurde ein Hitzeschock (37°C) induziert, und die Fluoreszenz wurde mit einem ClarioStar-Plattenlesegerät (BMG, Ortenberg, Deutschland) alle 30 Minuten über einen Zeitraum von 17 h gemessen. Zum Nachweis der grünen Fluoreszenz von SYTOXTM, wurde die Anregungswellenlänge auf 485 nm festgelegt und die Emission bei 538 nm detektiert.

### Beispiel 2:

Oxidative Phosphorylierung (OxPhosC) von aus dem Hippocampus transgener F344-AD Ratten isolierten Mitochondrien nach Behandlung

Nach Dekapitation der F344-AD Ratten wird der Hippocampus isoliert und bei 4°C homogenisiert. Nach Zentrifugationsschritten werden die isolierten Mitochondrien in die Kammer eines Respirometers gegeben. Durch nacheinander erfolgende Zugabe von Substraten und Inhibitoren der Atmungskette kann der Einfluss auf den Sauerstoffverbrauch in der Kammer an einer Platinelektrode gemessen werden und so einzelne Komplexe isoliert auf ihre Aktivität untersucht werden (ausgearbeitet von Tina M. Schwarzkopf, Konrad A. Koch, Jochen Klein, Neurodegeneration after transient brain ischemia in aged mice: Beneficial effects of bilobalide, Brain Research, Vol. 1529, 5 September 2013, Pages 178-187 mit weiteren Nachweisen).

| Legende | Behandlung | Dosierung |
|---|---|---|
| A, E (Control) | Keine Zusätze | 0 |
| B | Magnesiumorotat | 500 mg/kg |
| C | Benfotiamin | 300 mg/kg |
| D, F (Cocktail) | Magnesiumorotat | 500 mg/kg |
| | Benfotiamin | 300 mg/kg |
| | Vitamin B12 | 1 mg/kg |
| | Folsäure | 10 mg/kg |
| | Vitamin D3 | 3000 I.U./kg |

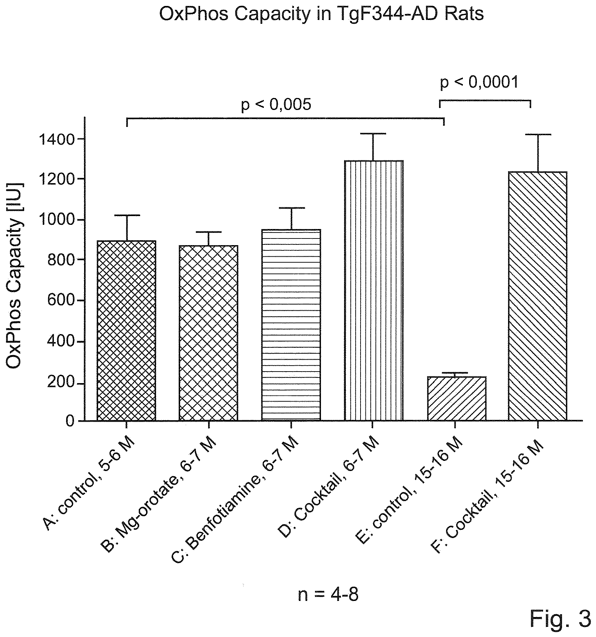

Die Ergebnisse sind in Figur 3 gezeigt.

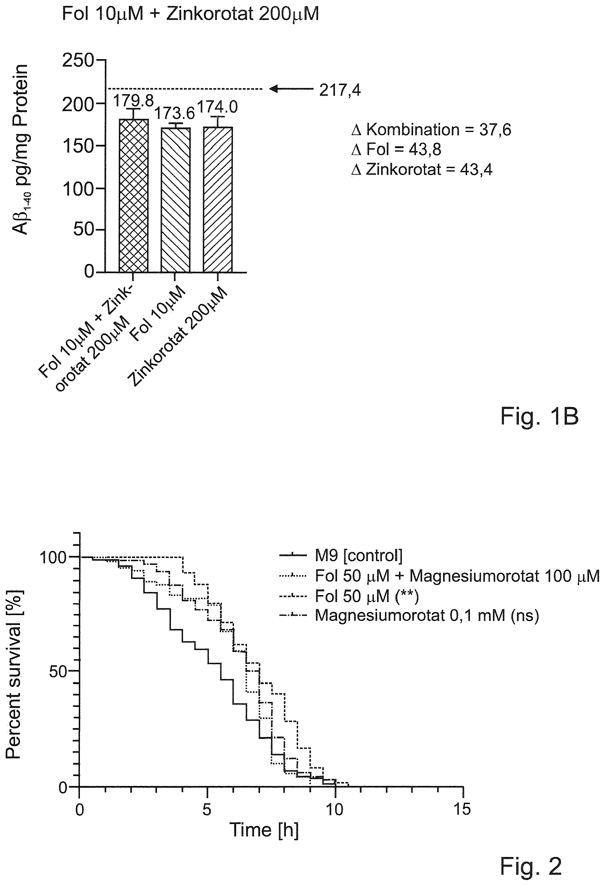

Figur 1 zeigt die Menge an Beta-Amyloid-Peptid (Aβ1-40) in pg/mg Protein in SH-SY5Y-APP695 nach 24h Inkubation mit den Wirkstoffen A.) Folsäure (Fol; 10 µM) und Magnesiumorotat (200 µM) sowie deren Kombination und B.) Folsäure (Fol; 10 µM) und Zinkorotat (200 µM) sowie deren Kombination. Es werden die jeweiligen Mittelwerte aus 6 unabhängigen Versuchen (n=6) sowie die dazugehörigen Standardfehler (± SEM) dargestellt. Die Signifikanzen (***p < 0.001 bzw. ****p < 0.0001) wurden mittels einer one-way ANOVA bestimmt. Die gepunktete Linie gibt den Mittelwert der Kontrolle an (217.4 pg/mg Aβ).

Aus Figur 1A ergibt sich ein synergetischer Effekt für Folsäure und Magnesiumorotat, da:
Δ Kombination > Δ Magnesiumorotat + Δ Fol.

Figur 2 zeigt die Lebensspannen von C. elegans im Hitzestress-Assay nach der Behandlung mit Magnesiumorotat und Folsäure ("Fol") und deren Kombination in Form von Kaplan-Meier-Kurven. Unbehandelte Würmer dienten als Kontrolle (M9 [control]). Es werden die jeweiligen Mittelwerte sowie die dazugehörigen Standardfehler (± SEM) dargestellt. Die Signifikanz (**p < 0.01) wurde mittels log-rank (Mantel-Cox) ermittelt Test errechnet.

Figur 3 zeigt die Kapazität (IU = pmol/min) der oxidativen Phosphorylierung (OxPhosC) von aus dem Hippocampus transgener F344-AD Ratten isolierten Mitochondrien. Auf der Abszisse sind die vier Behandlungen (B, C, D, F), sowie die zwei Altersgruppen (6-7 Monate, 15-16 Monate) aufgetragen. Zwischen den 6-7 Monate alten Tieren ergeben sich durch die Behandlungen keine signifikanten Unterschiede hinsichtlich der OxPhosC, wenngleich die Behandlung mit dem Cocktail (Gruppe D) eine Erhöhung der OxPhosC bewirkt. Altersbedingt nimmt die OxPhosC statistisch signifikant ab (vgl. Gruppe A mit Gruppe E). Dieser Effekt des Alters lässt sich durch die Behandlung mit dem Cocktail statistisch signifikant beeinflussen, bzw. aufheben (vgl. Gruppe E mit Gruppe F).

### Referenzen

1. Hardy J. A hundred years of Alzheimer's disease research. Neuron. 2006; 52(1):3-13.
2. Selkoe DJ and Hardy J. The amyloid hypothesis of Alzheimer's disease at 25 years. EMBO Mol Med. 2016; 8(6):595-608.
3. Querfurth HW and LaFerla FM. Alzheimer's disease. N Engl J Med. 2010; 362(4):329-344.
4. Harold D, Abraham R, Hollingworth P, Sims R, Gerrish A, Hamshere ML, Pahwa JS, Moskvina V, Dowzell K, Williams A, Jones N, Thomas C, Stretton A, Morgan AR, Lovestone S, Powell J, et al. Genome-wide association study identifies variants at CLU and PICALM associated with Alzheimer's disease. Nat Genet. 2009; 41(10):1088-1093.
5. Lambert JC, Ibrahim-Verbaas CA, Harold D, Naj AC, Sims R, Bellenguez C, DeStafano AL, Bis JC, Beecham GW, Grenier-Boley B, Russo G, Thorton-Wells TA, Jones N, Smith AV, Chouraki V, Thomas C, et al. Meta-analysis of 74,046 individuals identifies 11 new susceptibility loci for Alzheimer's disease. Nat Genet. 2013; 45(12):1452-1458.
6. Genin E, Hannequin D, Wallon D, Sleegers K, Hiltunen M, Combarros O, Bullido MJ, Engelborghs S, De Deyn P, Berr C, Pasquier F, Dubois B, Tognoni G, Fievet N, Brouwers N, Bettens K, et al. APOE and Alzheimer disease: a major gene with semidominant inheritance. Mol Psychiatry. 2011; 16(9):903-907.
7. Naj AC, Jun G, Beecham GW, Wang LS, Vardarajan BN, Büros J, Gallins PJ, Buxbaum JD, Jarvik GP, Crane PK, Larson EB, Bird TD, Boeve BF, Graff-Radford NR, De Jager PL, Evans D, et al. Common variants at MS4A4/MS4A6E, CD2AP, CD33 and EPHA1 are associated with late-onset Alzheimer's disease. Nat Genet. 2011; 43(5):436-441.
8. Daulatzai MA. Cerebral hypoperfusion and glucose hypometabolism: Key pathophysiological modulators promote neurodegeneration, cognitive impairment, and Alzheimer's disease. J Neurosci Res. 2017; 95(4):943-972.
9. Baglietto-Vargas D, Shi J, Yaeger DM, Ager R and LaFerla FM. Diabetes and Alzheimer's disease crosstalk. Neurosci Biobehav Rev. 2016; 64:272-287.
10. Wild S, Roglic G, Green A, Sicree R and King H. Global prevalence of diabetes: estimates for the year 2000 and projections for 2030. Diabetes Care. 2004; 27(5):1047-1053.
11. Abate G, Marziano M, Rungratanawanich W, Memo M and Uberti D. Nutrition and AGE-ing: Focusing on Alzheimer's Disease. Oxid Med Cell Longev. 2017; 2017:7039816.
12. Qizilbash N, Gregson J, Johnson ME, Pearce N, Douglas I, Wing K, Evans SJ and Pocock SJ. BMI and risk of dementia in two million people over two decades: a retrospective cohort study. Lancet Diabetes Endocrinol. 2015; 3(6) :431-436.
13. Cordain L, Eaton SB, Sebastian A, Mann N, Lindeberg S, Watkins BA, O'Keefe JH and Brand-Miller J. Origins and evolution of the Western diet: health implications for the 21st century. Am J Clin Nutr. 2005; 81(2) :341-354.
14. Nishida C, Uauy R, Kumanyika S and Shetty P. The joint WHO/FAO expert consultation on diet, nutrition and the prevention of chronic diseases: process, product and policy implications. Public Health Nutr. 2004; 7(1A) :245-250.
15. Janson J, Laedtke T, Parisi JE, O'Brien P, Petersen RC and Butler PC. Increased risk of type 2 diabetes in Alzheimer disease. Diabetes. 2004; 53(2) :474-481.
16. Kroner Z. The relationship between Alzheimer's disease and diabetes: Type 3 diabetes? Altern Med Rev. 2009; 14(4) :373-379.
17. Clarke JR, Lyra ESNM, Figueiredo CP, Frozza RL, Ledo JH, Beckman D, Katashima CK, Razolli D, Carvalho BM, Frazao R, Silveira MA, Ribeiro FC, Bomfim TR, Neves FS, Klein WL, Medeiros R, et al. Alzheimer-associated Abeta oligomers impact the central nervous system to induce peripheral metabolic deregulation. EMBO Mol Med. 2015; 7(2) :190-210.
18. Jais A, Einwallner E, Sharif O, Gossens K, Lu TT, Soyal SM, Medgyesi D, Neureiter D, Paier-Pourani J, Dalgaard K, Duvigneau JC, Lindroos-Christensen J, Zapf TC, Amann S, Saluzzo S, Jantscher F, et al. Heme oxygenase-1 drives metaflammation and insulin resistance in mouse and man. Cell. 2014; 158(1) :25-40.
19. Calay ES and Hotamisligil GS. Turning off the inflammatory, but not the metabolic, flames. Nat Med. 2013; 19(3) :265-267.
20. Graham LC, Harder JM, Soto I, de Vries WN, John SW and Howell GR. Chronic consumption of a western diet induces robust glial activation in aging mice and in a mouse model of Alzheimer's disease. Sci Rep. 2016; 6:21568.
21. Rodriguez-Moran M, Guerrero-Romero F2. Oral magnesium supplementation improves the metabolic profile of metabolically obese, normal-weight individuals: a randomized double-blind placebo-controlled trial. Arch Med Res. 2014 Jul;45(5) :388-93. doi: 10.1016/j.arcmed.2014.05.003. Epub 2014 May 11
22. MISCHOULON DAVID ET AL: "The role of folate in depression and dementia.",THE JOURNAL OF CLINICAL PSYCHIATRY 2007, Bd. 68 Suppl 10, 2007, Seiten 28-33
23. Wenzhang Wang, Fanpeng Zhao, Xiaopin Ma, George Perry, Xiongwei Zhu, Mitochondria dysfunction in the pathogenesis of Alzheimer's disease: recent advances, Molecular Neurodegeneration (2020) 15:30
24. Simon M. Bell, Katy Barnes, Matteo De Marco, Pamela J. Shaw, Laura Ferraiuolo , Daniel J. Blackburn, Annalena Venneri, Heather Mortiboys, Mitochondrial Dysfunction in Alzheimer's Disease: A Biomarker of the Future? Biomedicines 2021, 9, 63
25. Catrin Herpicha, Kristina Franz, Ursula Müller-Werdan, Mario Ost, Kristina Norman, Age-related fatigue is associated with reduced mitochondrial function in peripheral blood mononuclear cells, Exp. Gerontology 2021, Vol. 144

## Patentansprüche

1. Mittel umfassend Magnesiumorotat und Folsäure oder Folate oder Tautomere und Salze davon zur Verwendung in der Prophylaxe und Behandlung von Demenz.

2. Mittel zur Verwendung in der Prophylaxe und Behandlung von einer Demenz nach Anspruch 1 ausgewählt aus Alzheimer Demenz bzw. Alzheimer-Krankheit, Demenz bei Alzheimer-Krankheit mit frühem Beginn (Typ 2), Demenz bei Alzheimer-Krankheit mit spätem Beginn (Typ 1), Demenz bei Alzheimer-Krankheit mit atypischer oder gemischter Form, Vaskuläre Demenz, Demenz bei Lewy-Körper-Krankheit.

3. Mittel zur Verwendung in der Prophylaxe und Behandlung von einer Demenz nach einem der Ansprüche 1 oder 2, wobei das molare Verhältnis von Magnesiumorotat zu Folsäure 2-25:1, insbesondere 20:1 beträgt.

4. Mittel zur Verwendung in der Prophylaxe und Behandlung von einer Demenz nach einem der Ansprüche 1 bis 3, wobei das Mittel zusätzlich mindestens eine oder mehrere Substanzen ausgewählt aus der Gruppe Zinkorotat, Benfotiamin, Vitamin D und Vitamin B enthält.

5. Mittel zur Verwendung in der Prophylaxe und Behandlung von einer Demenz nach einem der Ansprüche 1 bis 4, wobei das Mittel zusätzlich mindestens eine oder mehrere Substanzen ausgewählt aus der Gruppe Zinkorotat, Vitamin D3, Thiamin, Vitamin B6, Vitamin B12 und Benfotiamin enthält.

6. Mittel zur Verwendung in der Prophylaxe und Behandlung von einer Demenz nach einem der vorhergehenden Ansprüche, wobei das Mittel ein Arznei- oder Nahrungsergänzungsmittel oder eine bilanzierte Diät ist.

7. Pharmazeutische Zubereitungen enthaltend ein Mittel zur Verwendung in der Prophylaxe und Behandlung von einer Demenz nach einem der Ansprüche 1 bis 6, ggfs. samt geeigneter Trägerstoffe, insbesondere in Form von Tropfen, Saft, Sirup, Tabletten, Dragees, Kapseln, Retard-Formulierungen, Infusionen, Salben, Emulsionen, Puder, Pulver.

8. Pharmazeutische Zubereitungen enthaltend ein Mittel zur Verwendung in der Prophylaxe und Behandlung von einer Demenz nach einem der Ansprüche 1 bis 7 samt Hilfs- und Zusatzstoffen.

## Claims

1. Agent comprising magnesium orotate and folic acid or folates or tautomers and salts thereof for use in the prophylaxis and treatment of dementia.

2. Agent for use according to claim 1 in the prophylaxis and treatment of dementia selected from Alzheimer's dementia or Alzheimer's disease, dementia in Alzheimer's disease with early onset (type 2), dementia in Alzheimer's disease with late onset (type 1), dementia in Alzheimer's disease, atypical or mixed type, vascular dementia, Lewy body dementia.

3. Agent for use according to any of claims 1 or 2, wherein the molar ratio of magnesium orotate to folic acid is 2-25:1, in particular 20:1.

4. Agent for use according to any of claims 1 to 3, wherein the agent additionally contains at least one or more substances selected from the group containing zinc orotate, benfotiamine, vitamin D and vitamin B.

5. Agent for use according to any of claims 1 to 4, wherein the agent additionally contains at least one or more substances selected from the group containing zinc orotate, vitamin D3, thiamine, vitamin B6, vitamin B12 and benfotiamine.

6. Agent for use according to any of the preceding claims, wherein the agent is a drug or a dietary supplement or a balanced diet.

7. Pharmaceutical preparations containing an agent for use according to any of claims 1 to 6, optionally together with suitable carrier substances, in particular in the form of drops, juice, syrup, tablets, sugar-coated tablets, capsules, sustained-release formulations, infusions, ointments, emulsions, or powder.

8. Pharmaceutical preparations containing an agent for use according to any of claims 1 to 7 together with excipients and additives.

## Revendications

1. Agent comprenant de l'orotate de magnésium et de l'acide folique ou des folates ou des tautomères et leurs sels pour une utilisation dans la prophylaxie et le traitement de la démence.

2. Agent pour une utilisation selon la revendication 1 dans la prophylaxie et le traitement de la démence choisie parmi la démence d'Alzheimer ou la maladie d'Alzheimer, la démence dans la maladie d'Alzheimer à début précoce (type 2), la démence dans la maladie d'Alzheimer à début tardif (type 1), la démence dans la maladie d'Alzheimer de type atypique ou mixte, la démence vasculaire, la démence à corps de Lewy.

3. Agent pour une utilisation selon l'une des revendications 1 ou 2, dans lequel le rapport molaire entre l'orotate de magnésium et l'acide folique est de 2-25:1, en particulier 20:1.

4. Agent pour une utilisation selon l'une des revendications 1 à 3, dans lequel l'agent contient en outre au moins une ou plusieurs substances choisies dans le groupe contenant l'orotate de zinc, la benfotiamine, la vitamine D et la vitamine B.

5. Agent pour une utilisation selon l'une des revendications 1 à 4, dans lequel l'agent contient en outre au moins une ou plusieurs substances choisies dans le groupe comprenant l'orotate de zinc, la vitamine D3, la thiamine, la vitamine B6, la vitamine B12 et la benfotiamine.

6. Agent pour une utilisation selon l'une des revendications précédentes, dans lequel l'agent est un médicament ou un complément alimentaire ou un régime équilibré.

7. Préparations pharmaceutiques contenant un agent utilisable selon l'une des revendications 1 à 6, éventuellement avec des substances porteuses appropriées, notamment sous forme de gouttes, de jus, de sirop, de comprimés, de comprimés enrobés de sucre, de capsules, de formulations à libération prolongée, d'infusions, d'onguents, d'émulsions ou de poudre.

8. Préparations pharmaceutiques contenant un agent pour une utilisation selon l'une des revendications 1 à 7, ainsi que des excipients et des additifs.
